(19) Europäisches Patentamt
European Patent Office
Office européen des brevets

(11) Publication number: **0 192 168**
**B1**

(12) **EUROPEAN PATENT SPECIFICATION**

(45) Date of publication of patent specification: **23.01.91**

(51) Int. Cl.$^5$: **C 12 Q 1/68** // C07H21/00

(21) Application number: **86101725.9**

(22) Date of filing: **11.02.86**

(54) **Solution-phase dual hybridization assay for detecting polynucleotide sequences.**

(30) Priority: **22.02.85 US 704130**

(43) Date of publication of application:
**27.08.86 Bulletin 86/35**

(45) Publication of the grant of the patent:
**23.01.91 Bulletin 91/04**

(84) Designated Contracting States:
**AT BE CH DE FR GB IT LI LU NL SE**

(56) References cited:
**EP-A-0 131 830**
**EP-A-0 139 489**
**EP-A-0 145 356**
**EP-A-0 159 719**
**GB-A-2 125 964**

**GENE, vol. 21, 1983, pages 77-85, NL; M. RANKI et al.: "Sandwich hybridization as a convenient method for the detection of nucleic acids in crude samples"**

(73) Proprietor: **MOLECULAR DIAGNOSTICS, INC.**
**400 Morgan Lane**
**West Haven, CT 06516 (US)**

(72) Inventor: **Dattagupta, Nanibhushan**
**470 Prospect Street**
**New Haven Ct 06511 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

**EP 0 192 168 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a novel way of running tests to determine the presence of particular nucleic acid sequences in test samples and to novel probes useful therefor.

The application of two non-overlapping DNA probes for hybridization has been disclosed in PCT patent application No. 83/01459, European patent application No. 0070687 and 0070685 (Homo). PCT No. 83/01459 and 0070687 disclose the application of two non-overlapping hybridization probes for the detection of a particular poynucleotide sequence in a test sample. One of the probes is fixed to a solid support prior to hybridization. Although this method eliminates the problem of electrophoretic separation of nucleic acids before hybridization, the process is slow because of the heterogeneous phases utilized.

European publication No. 0070685 discloses a homogeneous phase two probe assay with a non-radiative transfer method. This method needs sophisticated equipment to monitor hybridization. The background cannot be eliminated because of brownian motion, some nonspecific reactions, and because the concentration of the unhybridized probes present in solution is always very high compared to the hybridized probes.

The earlier filed but later published EP 159 719 describes a method of hybridization wherein the hybrid is attached to a matrix by forming a complex on the solid support.

An improved heterogenous system involving two probes, one of which is immobilized, is described in European Patent Application No. 130 515.

An improved heterogenous system involving two probes, one of which is immobilized, is described in European Patent Application No. 84 107 248 and Ranki et al, Gene *21* (1983) 77—85. The probes can be DNA, RNA, mixed nucleic acids or oligonucleotides. There are disclosed tests for particular nucleic acid sequences, such as that indicating sickle cell anemia, for example, by contacting the sample with two probes. The immobilized probe, otherwise identified as a separation probe, is immobilized on a support such as nitrocellulose. The other probe, identified as the detection probe, carries a label for ultimate assay. Both probes include different nucleic acid fragments, both complementary to a different portion of the test sequence if present in the test sample. The sample and probes are mixed, subjected to hybridizing conditions and, if the sample contains the right sequence, its nucleic acid will serve as a bridge between the two probes. Thereby the label of the labeled probe will become attached to the solid support. The support is removed and then "read" for the presence of the label.

The probes can be such that the label on the solid support will indicate either a positive or negative result with regard to the condition to be detected. In addition to sickle cell anemia, the test can be for any other genetic condition, e.g., thalassemia, Tay-Sachs, etc. An identical procedure can also be followed for the detection of bacteria or viruses in test samples.

While such process produces satisfactory results, it is desired to speed up the diagnostic process, without the disadvantages attending the homogeneous two probe assay noted hereinabove.

This and other objects and advantages are realized in accordance with the present invention pursuant to which there is provided a homogeneous hybridization method coupled with a hybrid separation system. This procedure enables hybridization to occur rapidly and eliminates the background problem by selectively separating out the hybrids from the solution. The method requires only common laboratory equipment to assay the post hybridization products.

The diagnostic process takes place homogeneously, i.e., in solution, and subsequently the separation probe is immobilized and, with it, the detection probe, if in fact hybridization has taken place. Moreover, the efficiency of the process of hybridization is higher in solution than in a hetereogeneous system.

This is accomplished by using a separation probe which also carries a reactive site capable of forming a stable covalent bond with a reaction partner. The reaction partner is provided in an immobilized form such as attached to a solid support. Accordingly, after hybridization, the solution is contacted with the immobilized reaction partner to permit formation of a stable bond with the reactive site in the separation probe, the immobilized reaction partner is separated from the solution and either the resulting separated immobilized fraction or the remaining solution, or both, is assayed for the presence of the detection probe.

The solid support can be Sephadex gel, agarose, nitrocellulose, paper, plastic, etc.

Preferably the detection probe is labeled with a detectable chemical group which can be radioactive, fluorescent, enzymatic or the like, and any of those of European Patent Application No. 131 830 is suitable.

The separation and detection probes are used as dilute aqueous solutions which can be combined with each other and the test sample simultaneous or in any desired order of steps, possibly with dilutions. By utilizing suitable conditions of ionic strength, pH and temperature, if the proper components are present, hybridization will occur very rapidly. Then the immobilized reaction partner is introduced and, after a suitable time to permit interaction of the reaction partner and separation probe, the immobile phase or fraction is removed, washed and the assay conducted, in known manner as described in European Patent Application No. 130 515.

### Reactive Site/Reactive Partner Pairs

Essentially any pair of substances can be used for this function which exhibits an appropriate affinity for interacting to form a stable bond, that is a coupling between the two which remains substantially intact

during the subsequent assay steps, principally separation and detection steps. The bond formed is a covalent bond.

As for example, the separation probe can be modified to have reactive

$—NH_2$

$—SH$

$—COOH$

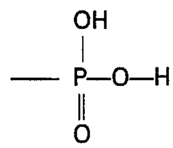

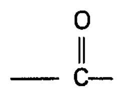

$—OH$

residues. This can be accomplished in known manner. Using 5-allylamino UTP or 8-hexyl amino ATP and terminal deoxynucleotidyl transferase $—NH_2$ residues can be introduced at the 3′ end of the separation probe. Using 4-thio UTP or 5-carboxy methyl UTP and TdT, —SH and —COOH residues can be introduced. Modified bases can also be introduced by nick translation. Alternatively a ligand can be covalently bound to the probe. The ligand can be the site of reaction. As for example a psorale an angelicin or azido ethidium with $—NH_2$ can be photochemically covalently bound to the probe and then modified via the reaction site in the ligand. A restriction enzyme digested fragment usually produces a 5′ -phosphorylated end. A carbonyl residue can be produced by oxidation of a terminal ribose residue (can be introduced via TdT reaction). All these site or sites can be present in one or multiple units per separation probe. Once these residues are available known reactions can be used to form covalent linkage between these residues and an immobilization medium e.g., solid particulate support having an —OH residue, or

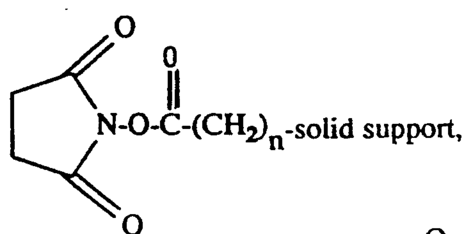

or HS-solid support, or 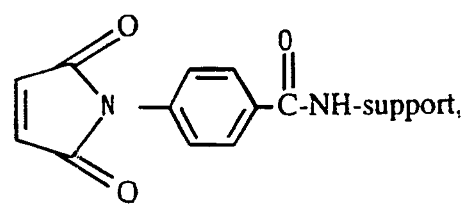

or 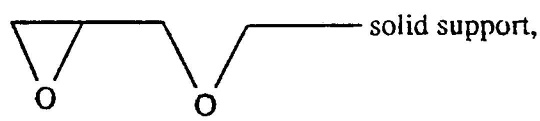

or OHC-solid suppon.

to form

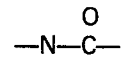

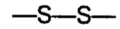

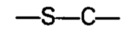

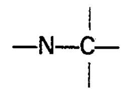

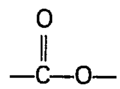

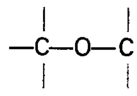

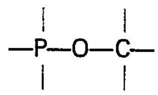

All these activated solid supports can be made by known reactions.

Immobilization

The reaction partner to the reactive site on the separation probe is used in the present assay in an immobilized form, that is, any suitable form that enables the reaction partner, and any components of the reaction mixture that have become associated with the reaction partner by hybridization and/or formation of the bond with the separation probe, to be subsequently isolated or separated from the remaining mixture such as by centrifugation, filtration, chromatography, or decantig. A variety of compositions and configurations of the immobilized reaction partner will thus be evident and available to the worker in the field. In general such include attachment to a solid support, polymerization or attachment to a solid support, hybridization or attachment to a water dispersible material which can be subsequently precipitated or aggregated.

It is particularly preferred to employ a solid support to which the reaction partner is attached or fixed by covalent bonds. The solid support can take a variety of shapes and compositions including microparticles, beds, porous and impermeable strips and membranes, the interior surface of reaction vessels such as test tubes and microtiter plates, and the like. Means for attaching a desired reaction partner to a selected solid support will be a matter of routine skill to the worker in the field.

Detection Systems

There are a variety of methods that can be used in the present invention for determining the presence of the detection probe in the separation immobilized fraction or in the remaining reaction solution in order to conclude the assay. One of ordinary skill in the art can choose from any conventional means for detecting the occurrence of hybridization between the detection probe and the sequence to be detected and the resulting presence of the detection probe in the immobilized phase or its reduced presence in the reaction mixture. In general, the detection step will be based on the use of a labeled form of the detection probe, the use of the detection probe that forms a uniquely detectable hybrid with the sequence of interest, or via secondary reactions which can only be carried out when hybridization takes place, e.g., primer extension reaction.

A particularly preferred approach to the detection step involves the use of a labeled form of the detetion probe. The label will be a native characteristic of the polynucleotide comprised in the probe or a substance which has a detectabble physical, chemical, or electrical property. When a detectable labeling substance is introduced, it can be linked directly such as by covalent bonds to the probe or can be linked indirectly such as by incorporation of the ultimately detectable substance in a microcapsule or liposome which in turn is linked to the detectable probe.

Labeling materials have been well-developed in the field of immunoassays and in general most any label useful in such methods can be applied to the present invention. Particularly useful are enzymatically active groups, such as enzymes (see Clin. Chem. (1976)22:1232, U.S. Reissue Pat. No. 31,006, and UK Pat. 2,019,408), enzyme substrates (see U.S. Pat. No. 4,492,751), coenzymes (see U.S. Pat. Nos. 4,230,797 and 4,238,565), and enzyme inhibitors (see U.S. Pat. No. 4,134,792); fluorescers (see Clin. Chem. (1979)25:353); chromophores; luminescers such as chemiluminescers and bioluminescers (see U.S. Pat. No. 4,380,580); specifically bindable ligands such as biotin (see European Pat. Spec. 63,879) or a hapten (see PCT Publ.

83—2286); and radioisotopes such as $^{3}H$, $^{35}S$, $^{32}P$, $^{125}I$, and $^{14}C$. Such labels are detected on the basis of their own physical properties (e.g., fluorescers, chromophores and radioisotopes) or their reactive or binding properties (e.g., ligands, enzymes, substrates, coenzymes and inhibitors). For example, a cofactor-labeled species can be detected by adding the enzyme (or enzyme where a cycling system is used) for which the label is a cofactor and a substrate or substrates for the enzyme. A hapten or ligand (e.g., biotin) labeled species can be detected by adding an antibody to the hapten or a protein (e.g., avidin) which binds the ligand, tagged with a detectable molecule. Such detectable molecule can be some molecule with a measurable physical property (e.g., fluorescence or absorbence) or a participant in an enzyme reaction (e.g., see above list). For example, one can use an enzyme which acts upon a substrate to generate a product with a measurable physical property. Examples of the latter include, but are not limited to, β-galactosidase, alkaline phosphatase and peroxidase.

Methods for preparing a label detection probe used in a preferred embodiment of the present invention are readily available from the prior art. When labeling probes one will employ synthetic approaches which are effective for modifying nucleic acids without substantially interfering with the ability of the labeled probe to participate in hybridization, and will select labels which are sufficiently stable under the conditions to be used for hybridization to enable their subsequent detection. Single stranded or double stranded regions of the probe can be labeled as desired.

By way of example, the following approaches can be used in labeling probes. Radiolabeled nucleotides can be incorporated into DNA probes by methods such as nick translation and terminal labeling with terminal deoxynucleotidyl transferase. Radiolabeled nucleotides can be incorporated into RNA probes during in vitro synthesis with DNA dependent RNA polymerase from bacteriophage SP6 using the Riboprobe$^{RM}$ DNA template system from Promega Biotec, Madison, WI. The method of Langer et al [(1981) Proc. Nat'l. Acad. Sci., 78:6633] can be used to couple biotin to the primary amine of 5-(3-amino)allyluride and deoxyuridine triphosphates. These biotinylated nucleotides can be incorporated into double stranded DNA by nick translation or added to the 3'-OH terminus with terminal deoxynucleotidyl transferase. Biotin can also be attached to the 3'-OH terminus of RNA through polyamine [Broker, T. R., (1978) Nucl. Acids Res. 4:363] and cytochrome C bridges [Sodja, A. and Davidson, N. (1978) Nucl. Acids. Res. 5:385]. Direct coupling of protein labels to probes can be accomplished by the method of Renz [(1982) EMBO Journal, 2:817] who coupled $^{125}I$-histones to denatured DNA with glutaraldehyde. Enzymes such as peroxidase and alkaline phosphatase can be linked to DNA probes by means of similar chemistry [Renz and Kurz (1984) Nucl. Acids Res. 12:3435]. Other chemistries for end-labeling DNA probes include that described by Eshaghpour et al [(1979) Nucl. Acids Res. 7:1485]. One or more 4-thiouridine residues can be introduced on the 3'-OH ends of DNA and the thiols reacted with various electrophilic low molecular weight reagents. This chemistry can be used to attach various haptens to DNA probes. Labeling with the hapten N-acetoxy-N-2-acetylaminofluorene is described by Tchen et al [(1984) Proc. Nat'l. Acad. Sci. 81:3466]. DNA and RNA probes can be reacted with N-acetoxy-N-2-acetylaminofluorene to yield an adduct having N-2-acetylaminofluorene residues attached at the 8-carbon of guanine. The covalently modified DNA can be detected with antibody raised against the N-acetoxy-N-2-acetyl-aminofluorene residue. The method of Hu and Messing [(1982) Gene, 17:271) can be used for adding labels to probes cloned into single stranded M13 vectors. A universal primer, complementary to the region 5' to the cloning site, initiates DNA synthesis complementary to the M13 strand downstream from the probe sequence. Since the DNA polymerase will incorporate radioactive nucleotide triphosphates and biotin 5-(3-aminoallyl) deoxyuridine triphosphate into the new strand, those labels can be attached to the vector away from the probe sequence. The double stranded portion can also be modified by reaction with 8-azidoethidium.

Another particularly preferred approach to the detection step involves the use of a detection probe system wherein the hybrid formed between the polynucleotide sequence of interest and the detection probe is antigenically distinct from its individual single strands. One is thus enabled to detect the presence of the detection probe in the immobilized fraction containing hybridized detection probe by adding an antibody reagent as discussed above that is selective for binding such hybrids. Preferred antibody reagents will be those that are selective for binding double stranded nucleic acids over single stranded nucleic acids, e.g., those which selectively bind (i) DNA·RNA or RNA·RNA hybrids or (ii) intercalation complexes. In the first instance, an antibody reagent selective for binding DNA·RNA hybrids will be useful where one of the detection probe and the sequence to be detected is DNA and the other is RNA, and in either case of course the separation probe will be RNA or DNA the same as the sequence to be detected. One can use an antibody reagent selective for finding RNA·RNA hybrids where both the detection probe and the sequence of interest are RNA and the separation probe is DNA. In the case of intercalation complexes, the assay will be designed so that the hybrids formed between the detection probe and the sequence of interest will comprise a nucleic acid intercalator bound thereto in the form of intercalation complexes.

Immunogens for stimulating antibodies specific for RNA·DNA hybrids can comprise homopolymeric or heteropolymeric polynucleotide duplexes. Among the possible homopolymer duplexes particularly preferred is poly(rA)·poly(dT) [Kitagawa and Stollar (1982) Mol. Immuno. 19:413]. However, in general heteropolymer duplexes will be preferably used and can be prepared in a variety of ways, including transcription of φX174 virion DNA with RNA polymerase [Nakazato (1980) Biochem. 19:2835]. The selected RNA·RNA duplexes are adsorbed to a methylated protein, or otherwise linked to a conventional immunogenic carrier material, such as bovine serum albumin, and injected into the desired host animal

[see also Stollar (1980) Meth. Enzymol. 70:70]. Antibodies to RNA·RNA duplexes can be raised against double stranded RNAs from viruses such as reovirus or Fiji disease virus which infects sugar cane, among others. Also, homopolymer duplexes such as poly(rI)·poly(rC) or poly(rA)·poly(rU), among others, can be used for immunization as above.

Antibodies to intercalation complexes can be prepared against an immunogen which will usually comprise an ionic complex between a cationic protein or protein derivative (e.g., methylated bovine serum albumin) and the anionic intercalator-nucleic acid complex. Ideally, the intercalator will be covalently coupled to the double stranded nucleic acid. Alternatively, the intercalator-nucleic acid conjugate can be covalently coupled to a carrier protein. The nucleic acid portion of the immunogen can comprise the specific paired sequences found in the assay hybrid or can comprise any other desirable sequences since the specificity of the antibody will generally not be dependent upon the particular base sequences involved.

In other instances where an antibody reagent selective for intercalation complexes is employed in the detection system, a variety of intercalator compounds can be involved. In general it can be said that the intercalator compound preferably is a low molecular weight, planar, usually aromatic but sometimes polycyclic, molecule capable of binding with double stranded nucleic acids, e.g., DNA·DNA, DNA·RNA, or RNA·RNA duplexes, usually by insertion between base pairs. The primary binding mechanism will usually be noncovalent, with covalent binding occurring as a second step where the intercalator has reactive or activatable chemical groups which will form covalent bonds with neighboring chemical groups on one or both of the intercalated duplex strands. The result of intercalation is the spreading of adjacent base pairs to about twice their normal separation distance, leading to an increase in molecular length of the duplex. Further, unwinding of the double helix of about 12 to 36 degrees must occur in order to accommodate the intercalator. General reviews and further information can be obtained from Lerman, J. Mol. Biol. 3:18(1961); Bloomfield et al. "Physical Chemistry of Nucleic Acids", Chapter 7, pp. 429—476, Harper and Rowe, NY(1974); Waring, Nature 219—1320 (1968); Hartmann et al, Agnew. Chem., Engl. Ed. 7:693(1968); Lippard, Accts. Chem. Res. 11:211(1978); Wilson, Intercalation Chemistry (1982), 445; and Berman et al, Ann. Rev. Biophys. Bioeng. 20:87(1981). Exemplary of intercalators are acridine dyes, e.g., acridine orange, the phenanthridines, e.g., ethidium, the phenazines, furocoumarins, phenothiazines, and quinolines.

The intercalation complexes are formed in the assay medium during hybridization by use of a detection probe which has been modified in its complementary, single stranded region to have the intercalator chemically linked thereto such that upon hybridization the intercalation complexes are formed. Essentially any convenient method can be used to accomplish such linkage. Usually, the linkage is formed by effecting intercalation with a reactive, preferably photoreactive intercalator, followed by the linking reaction. A particularly useful method involves the azidointercalators. Upon exposure to ultraviolet or visible light, the reactive nitrenes are readily generated. The nitrenes of arylazides prefer insertion reactions over their rearrangement products [see White et al. Methods in Enzymol. 46:644(1977)]. Representative azidointercalators are 3-azidoacridine, 9-acizoacridine, ethidium monoazide, ethidium diazide, ethidium dimer azide [Mitchell et al, JACS 104:4265(1982)] 4-azido-7-chloroquinoline, and 2-azidofluorene. Other useful photoreactable intercalators are the furocoumarins which form [2 + 2] cycloadducts with pyrimidine residues. Alkylating agents can also be used such as bischloroethylamines and epoxides or aziridines, e.g., aflatoxins, polycyclic hydrocarbon epoxides, mitomycin, and norphillin A. The intercalator-modified duplex is then denatured to yield the modified single stranded probe.

The detection of antibody reagent that binds to the antigenically distinct hybrid formed between the detection probe and the sequence of interest can proceed in any conventional manner. For example, one can employ antibody reagent which has been labeled with any detectable chemical group as discussed above. The preparation of labeled antibodies is described extensively in the literature. Incorporation of $^{125}$I-label can be accomplished by the method of Bolton and Hunter (1972) Biochem. J. 133:529. Ishikawa et al (1982) J. Immunoassay 4:209 have outlined several different methods for coupling various enzymes to antibodies. Yoshitake et al (1979) Eur. J. Biochem. 101:395 have described a method for using maleimides to couple glucose oxidase to antibody. Alkaline phosphatase can be coupled to antibody with glutaraldehyde (Voler et al (1976) Bull. World Health Organ., 53:55]. Antibodies can be labeled with fluorescein by the method of Blakeslee and Baines (1976) J. Immunol. Meth., 13:305. Chemiluminescent labels can be introduced by the method of Schroeder et al (1981) Clin. Chem. 27:1378. Alternatively, the antibody reagent can be detected based on a native property such as its own antigenicity. A labeled anti-(antibody) antibody will bind to the primary antibody reagent where the label for the second antibody is any conventional label as above. Further, antibody can be detected by complement fixation or the use of labeled protein A, as well as other techniques known in the art for detecting antibodies.

Reaction Mixture

The test sample to be assayed can be any medium of interest, and will usualy be a liquid sample of medical, veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swab, and nasopharnygal aspirates. Where the test sample obtained from the patient or other source to be tested contains principally double stranded nucleic acids, such as contained in cells, the sample will be treated to denature the nucleic acids, and if necessary first to release acids from cells.

Denaturation of nucleic acids is preferably accomplished by heating in boiling water or alkali treatment (e.g., 0.1 N sodium hydroxide), which if desired, can simultaneously be used to lyse cells. Also, release of nucleic acids can, for example, be obtained by mechanical disruption (freeze/thaw, abrasion, sonication), physical/chemical disruption (detergents such as Triton, Tween, sodium dodecylsulfate, alkali treatment osmotic shock, or heat), or enzymatic lysis (lysozyme, proteinase K, pepsin). The resulting test medium will contain nucleic acids in single stranded form which can then be assayed according to the present hybridization method. Additionally, the sample nucleic acids can be fragmented specifically or nonspecifically in order to conduct a particular desired assay such as where point mutations are detected by specific endonuclease treatment followed by dual hybridization restriction (see for example, European Application No. 130 515).

As is known in the art, various hybridization conditions can be employed in the assay. Typically, hybridization will proceed at slightly elevated temperatures, e.g., between about 35 and 75°C and usually around 65°C, in a solution comprising buffer at pH between about 6 and 8 and with appropriate ionic strength (e.g., 5XSSC where 1XSSC = 0.15M sodium chloride and 0.015M sodium citrate, pH 7.0) and optionally protein such as bovine serum albumin, and a denatured foreign DNA such as from calf thymus or salmon sperm. In cases where lower hybridization temperatures are desirable, hydrogen bonding reagents such as dimethylsulfoxide and formamide can be included. The degree of complementarity between the sample and probe strands required for hybridization to occur depends on the stringency of the conditions. Factors which determine stringency are known in the art.

Normally, the temperature conditions selected for hybridization will be incompatible with the binding of antibody reagent to formed hybrids and detection of the label response. Accordingly, any antibody reagent binding step and label detection step will proceed after completion of the hybridization step. The reaction mixture will usually be brought to a temperature in the range of from about 3°C to about 40°C and the binding and detection steps then performed. Dilution of the hybridization mixture prior to addition of antibody reagent is desirable when the salt and/or formamide concentrations are high enough to interfere significantly with the antibody reagent is desirable when the salt and/or formamide concentrations are high enough to interfere significantly with the antibody binding reaction. In the case of assays which involve the use of label binding partners or labeled antibody reagent to detect hybridization of the detection probe, the sequence of assasy steps will generally proceed as follows. The hybridization reactions will be first accomplished with the test sample commonly having been pretreated as discussed above. The two probes can be contacted with the test sample simultaneously or in sequence as desired. The immobilization and the contact of the labeled binding partner or antibody reagent can then be performed simultaneously or in either sequence. Finally, the label will be measured in the immobilized fraction or the remaining reaction mixture. Variations in these steps will be evident to one working in the art.

Reagent System

The present invention additionally provides a reagent system, i.e., reagent combination or means, comprising all of the essential elements required to conduct a desired assay method. The reagent system is presented in a commercially packaged form, as a composition or admixture where the compatibility of the reagents will allow, in a test device configuration, or more usually as a test kit, i.e., a packaged combination of one or more containers, devices, or the like holding the necessary reagents, and usually including written instructions for the performance of assays. Reagent system of the present invention include all configurations and compositions for performing the various hybridization formats described herein.

In all cases, the reagent system will comprise (1) the firt, separation probe as described herein, (2) the second, detection probe as described herein, and (3) the immobilized reaction partner. A test kit form of the system can additionally include ancillary chemicals such as the components of the hybridization solution and denaturation agents capable of converting double stranded nucleic acids in a test sample into single stranded form.

EXAMPLES

Use of solution hybridization and separation of hybrid for the detection of sickle cell anemia

Steps:
1. Preparation of reactive separation probe
2. Labeling of detection probe
3. Preparation of support for immobilization of the hybrid
4. Hybridization and separation of the hybrid and assay

Methods of collection of patient's blood sample, isolation of test DNA, digestion of the test sample have been described in detail by Wilson et al U.S. Patent No. 4,395,486. The parent plasmid for the preparation of probe b BR 322Pst (4.4K6) is also described in that patent.

1. Preparation of the reactive separation probe

1 mg of p BR 322 b Pst is digested with AluI and 737 base pair fragment is isolated from that digest according to Wilson et al (U.S. Pat. No. 4,395,486). The 737 b.p. fragment is then digested further with the enzyme DdeI and the fragments 201 and 175 (base pair long) are separated and isolated from 4%

polyacrylamide gel. The 201 b.p. fragment is used as the separation probe and 175 b.p. fragment is used as the detection probe. As has been described before sickle cell mutation is at the junction of the two fragments (U.S. Pat. No. 4,395,486). Hybridization of both probes to a single piece of DNA fragment of size 376 b.p. produced by Ddel digestion will indicate sickle mutation.

The invention will be further described in the following illustrative example with reference to the accompanying drawing which is a flow sheet of a process in accordance with the invention.

10 µg of 201 b.p fragment is dissolved in 0.1 ml 10 mM borate buffer (pH 8.6) (separation probe 10). 1 µl (1 mg/ml) of aqueous solution of 4'-aminomethyl-4,5'-dimethylangelicin is added and the mixture is irradiated at 346 nm for 15 minutes (separation probe A). Then 10 µl (1 mg/ml in dimethyl formamide) of N-hydroxysuccinimido biotin is added as the biootinylation agent 12. The mixture is left at room temperature for 16 hours. The reaction mixture is dialyzed extensively against 10 mM tris HCl and 1 mM EDTA (pH 7.2) buffer. The sample is then further purified by precipitation with ethanol. The solid is redissolved in 100 µl tris-EDTA buffer. 1 µl of this solution is assayed for biotin using a kit purchased from Bethesda Research Laboratory, Gaithersburg MD. This is biotinylated separation probe 14 (separatoin probe B).

2. Labeling of the detection probe

The detection probe is the 175 b.p. fragment. The 175 b.p. fragment is labeled with label 18 by nick translation (4,395,486). Using $^{32}P$ labeled deoxy NTP's 16 instead of cold NTP, a labeled detection probe 20 with high $^{32}P$ specific activity is synthesized. This 175 b.p. fragment is a non-overlapping specific fragment for 201 b.p. separation probe for the detection of b globin gene mutation.

3. Immobilization of Streptavidin

Commercially available streptavidin (BRL) is immobilized to agarose 22 by a known method. (Cuatrecasas and Parikh — Biochemistry 11, 2291 (1972)). After immobilization it is kept soaked in large excess of herring sperm DNA solution in 1 mM tris 0.1 mM EDTA (pH ∾ 7).

4. Detection of Sickle Cell DNA in blood

It is known that the restriction enzyme Ddel digests wild type (normal) and sickle cell DNA differently. This polymorphism can be detected by the present method. If both the separation probe 14 (Example 1) and detection probe 20 (Example 2) hybridize, the test DNA has not been recognized by Ddel at the specific site of determination. This will indicate that the test DNA 24 has a sequence of sickle cell genome. Under only one condition both separation and detection probes will hybridize side by side to a linear piece of DNA.

From 10 ml blood sample, DNA is isolated, digested with the restriction enzyme (2 units of Ddel/ug of DNA) in a known manner (U.S. Patent No. 4,395,486). The digested DNA is deproteinized by phenol extraction, then dialyzed against 10 mM tris 1 mM EDTA buffer.

Use of separation probe A with A with primary amine

Since the probe after photochemical reaction with 4'-aminomethyl-4,5'dimethylangelicin

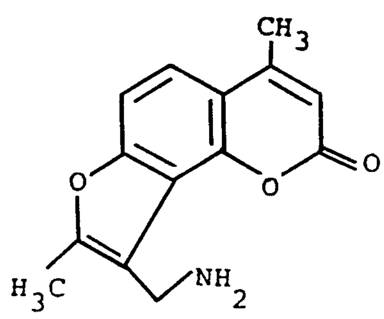

will contain primary —$NH_2$ residues they can be directly coupled to a solid support containing

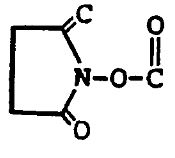 residue

(cuatrecasas of Parikh Biochemistry 11, 2291 (1972))

2 µg biotinylated separation probe, 0.2 µg $^{32}P$ labeled detection probe and the DNA extraction from blood are mixed in 10 mM tris 1 mM EDTA buffer, final total volume 2 ml. The solution is heated to 95°C, then incubated at 65°C for 15 minutes. Then it is chilled in ice and 20 ml water is added to reduce the ionic

strength. This process is done to reduce the Tm so that non-specific hybrids will melt at 30°C. Since the binding constant of biotin to streptavidin is high, dilution to this level does not create any problem of ligand-protein interaction. After dilution, the solution is incubated at 30°C for 15 minutes and then 1 ml agarose-streptavidin 22 (Example 3) in swollen conditon is added, stirred and centrifuged. The solid is then washed at room temperature 5 times with 1 mM tris 0.1 mM EDTA. The solid is then taken in a vial and counted in a scintillation counter for $^{32}P$. This solid can be used for autoradiographic detection in usual manner.

As has been described above, if there is any radioactivity above the background level on the bead, the DNA specimen is originated from a sickle cell patient or a carrier. If there is no radioactivity present on the beads, the DNA is from a normal blood sample.

The temperature of hybridization, salt conditions and buffers can be varied. The specific conditions of hybridization are dependent upon the type of nucleic acids, length, sequence, size of the probe, etc. Instead of streptavidin, an antibody against biotin can be used for separation. Instead of biotin labeling other haptens, ligands or an oxidizable residue can be used as binder to the solid support. The label on the detection probe can be, for example, a ligand, fluorophore or enzyme which can be assayed in known manner. Instead of using a Streptavidin coupled support of N-hydroxysuccinimide activated agarose is used the hybrid will form a covalent amide linkage via separation probe.

It will be understood that the specification and examples are illustrative but not limitative of the present invention and that other embodiments within the spirit and scope of the invention will suggest themselves to those skilled in the art.

**Claims**

1. A method of detecting the presence of a particular polynucleotide sequence in a test sample, comprising the steps of contacting the test sample with a first, separation nucleic acid probe and a second, detection nucleic acid probe, said probes each comprising at least one single stranded base sequence that is hybridizable with a mutually exclusive portion of the sequence to be detected, the separation probe comprising additionally a reactive chemical group capable of forming a stable covalent bond to a reactive solid support, such contact between the test sample and probes being effected in solution under hybridizing conditions, contacting the resulting solution with the reactive solid support under conditions to form said stable covalent bond with the reactive chemical group in the separation probe, separating the resulting immobilized fraction from the remaining solution, and determining the presence of the detection probe in the separated immobilized fraction or in the remaining solution.

2. The method of Claim 1 wherein the reactive chemical group in the separation probe is selected from $-NH_2$, —SH, —COOH,

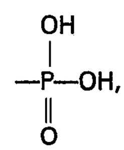 , 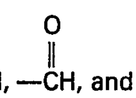 , and —OH.

3. The method of claim 2 wherein the reactive solid support is selected from

HO-support,

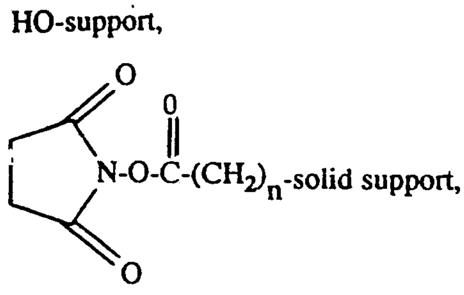

HS-solid support,

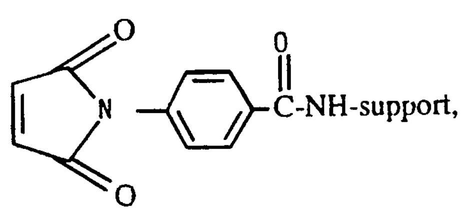

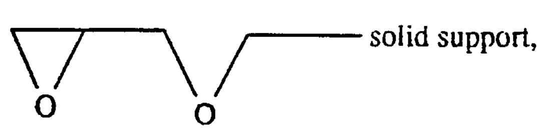

or OHC-solid support.

4. The method of any of Claims 1 to 3 wherein the detection probe comprises a detectable label and

wherein the presence of the detection probe in the separated immobilized fraction or the remaining solution is determined by measuring such label therein.

4. The method of Claim 4 wherein the detectable label is an enzymatically active group, a fluororescer, a chromophore, a luminescer, a radioisotope or a specifically bindable ligand which can be determined by binding with a labeled binding partner therefore.

6. The method of any of Claims 1 to 5 wherein the hybrid formed between the polynucleotide sequence of interest and the detection probe is antigenically distinct from its individual strands and wherein the presence of the detection probe in the separated immobilized fraction is determined by binding with an antibody reagent that is selective for binding said hybrid and measurement of the antibody reagent that becomes bound to such separated immobilized fraction.

7. A reagent combination for detecting the presence of a particular polynucleotide sequence in a test sample, comprising (1) a first, separation probe (2) a second detection probe, said probes each comprising at least one single stranded base sequence that is hybridizable with a mutually exclusive portion of the sequence to be detected, the separation probe comprising additionally a reactive chemical group capable of forming a stable covalent bond to a reactive solid support, and (3) the reactive solid support, wherein the reactive chemical group in the separation probe is selected from $—NH_2$, —SH, —COOH,

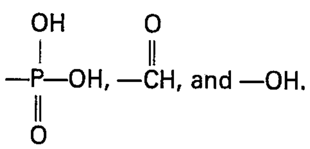

and wherein the reactive solid support is selected from

HO-support,

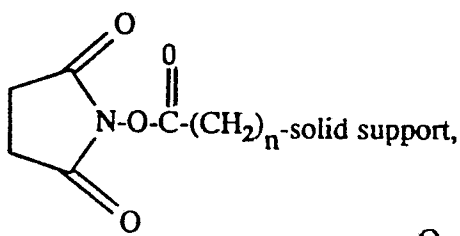

HS-solid support,

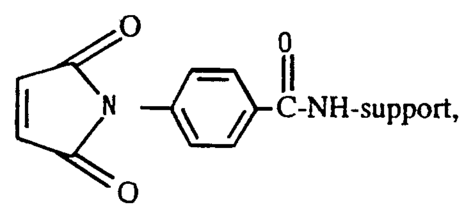

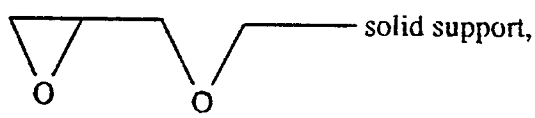

or OHC-solid support.

8. Use of the reagent combination of claim 7 for detecting the presence of a particular polynucleotide sequence in a test sample.

**Patentansprüche**

1. Verfahren zum Nachweis einer speziellen Polynukleotidsequenz in einer Probe, das die Schritte umfasst, dass man die Testprobe mit einer ersten Nukleinsäure-Separationssonde und einer zweiten Nukleinsäure-Detektionssonde in Berührung bringt, wobei jede dieser Sonden wenigstens eine einzelsträngige Basensequenz, die hybridisierbar mit einem sich gegenseitig ausschliessenden Teil der nachzuweisenden Sequenz ist, umfasst, wobei die Separationssonde zusätzlich eine reaktive chemische Gruppe, die sich dazu eignet, eine stabile kovalente Bindung an einem reaktiven festen Träger zu bilden,

umfasst, wobei ein solcher Kontakt zwischen der Testprobe und den Sonden in Lösung unter Hybridisierungsbedingungen bewirkt wird, und man die sich ergebende Lösung mit em reaktiven festen Träger unter Bedingungen in Berührung bringt, bei denen die stabile kovalente Bindung mit der reaktiven chemischen Gruppe in der Separationssonde gebildet wird und dass man die sich ergebende immobilisierte Fraktion aus der zurückbleibenden Lösung abtrennt und das Vorhandensein der Detektionssonde in der getrennten immobilisierten Fraktion oder in der zurückbleibenden Lösung bestimmt.

2. Verfahren nach Anspruch 1, bei dem die reaktive chemische Gruppe in der Separationssonde aus —$NH_2$, —SH, —COOH,

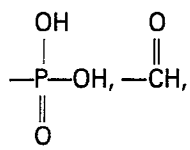, und —OH.

ausgewählt ist.

3. Verfahren nach Anspruch 2, bei dem der reaktive feste Träger aus

OH-Träger, 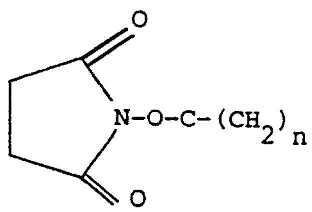 -fester Träger, HS-fester Träger,

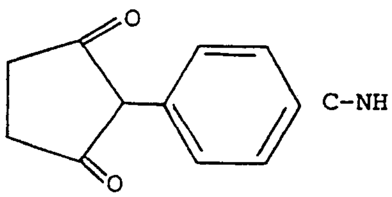 -Träger, 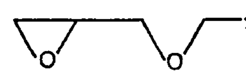fester Träger

oder OHC-fester Träger ausgewählt ist.

4. Verfahren nach einem der Ansprüche 1 bis 3, wobei die Detektionssonde eine nachweisbare Markierung enthält und das Vorhandensein de Detektionssonde in der abgetrennten immobilisierten Fraktion oder der zurückbleibenden Lösung bestimmt wird, indem die Markierung darin gemessen wird.

5. Verfahren nach Anspruch 4, bei dem die nachweisbare Markierung eine enzymatisch aktive Gruppe, eine fluoreszierende Gruppe, ein Chromophor, eine lumineszierende Gruppe, ein Radioisotop oder ein spezifisch bindender Ligand, der deshalb durch Bindung an einen markierten Bindungspartner bestimmt werden kann, ist.

6. Verfahren nach einem der Ansprüche 1 bis 5, bei dem das zwischen der interessierenden Polynukleotidsequenz und der Detektionssonde gebildete Hybrid in seinen einzelnen Strängen antigenisch charakteristisch ist, und bei dem das Vorhandensein der Detektionssonde in der abgetrennten immobilisierten Fraktion durch Bindung an ein Antikörperreagens, das selektiv im Hinblick auf die Bindung dieses Hybrids ist, und durch Messung des Antikörperreagenses, das an eine solche abgetrennte, immobilisierte Fraktion gebunden wird, bestimmt wird.

7. Reagenskombination zum Nachweis des Vorhandenseins einer speziellen Polynukleotidsequenz in einer Testprobe, die umfasst: (1) eine erste Separationssonde, (2) eine zweite Detektionssonde, wobei jede dieser Sonden wenigstens eine einzelsträngige Basensequenz, die hybridisierbar mit einem sich gegenseitig ausschliessenden Teil der nachzuweisenden Sequenz ist, umfasst, und wobei die Separationssonde zusätzlich eine reaktive chemische Gruppe, die zur Bildung einer stabilen kovalenten Bindung an einen reaktiven festen Träger geeignet ist, umfasst, und (3) den reaktiven festen Träger, wobei die reaktive chemische Gruppe in der Separationssonde aus —$NH_2$, —SH, —COOH,

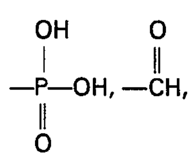, und —OH

ausgewählt ist, und wobei der reaktive feste Träger aus

OH-Träger, 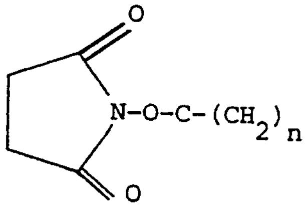 -fester Träger, HS-fester Träger,

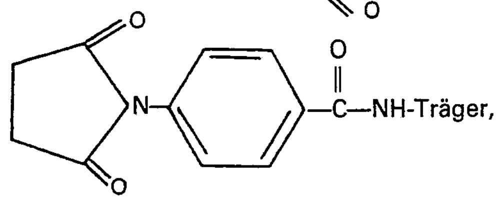

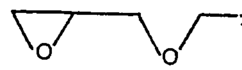 fester Träger

oder OHC-fester Träger ausgewählt ist.

8. Verwendung der Reagenskombination nach Anspruch 7 zum Nachweis des Vorhandenseins einer speziellen Polynukleotidsequenz in einer Testprobe.

**Revendications**

1. Procédé de détection de la présence d'une séquence polynucléotidique particulière dans un échantillon d'essai, comprenant les étapes de contact de l'échantillon d'essai avec une première sonde d'acide nucléique de séparation et une seconde sonde d'acide nucléique de détection, lesdites sondes comprenant chacune au moins une séquence de bases monocaténaire qui est hybridable avec une portion à exclusion réciproque de la séquence à détecter, la sonde de séparation comprenant de plus un groupe chimique réactif capable de former une liaison covalente stable avec un support solide réactif, le contact entre l'échantillon d'essai et les sondes étant effectué en solution dans des conditions d'hybridation, contact de la solution obtenue avec le support solide réactif dans des conditions formant ladite liaison covalente stable avec le groupe chimique réactif de la sonde de séparation, séparation de la fraction immobilisée obtenue d'avec la solution restante et détermination de la présence de la sonde de détection dans la fraction immobilisée séparée ou dans la solution restante.

2. Procédé selon la revendication 1, dans lequel le groupe chimique réactif dans la sonde de séparation est choisi parmi $—NH_2$, —SH, —COOH,

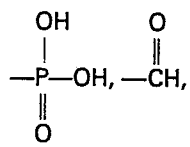, et —OH.

3. Procédé selon la revendication 2, dans lequel le support solide réactif est choisi parmi

HO-support solide,

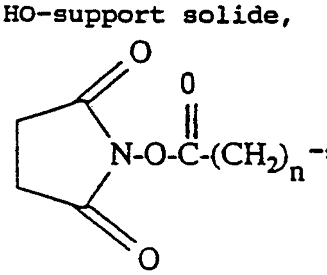 -support solide,

HS-support solide, 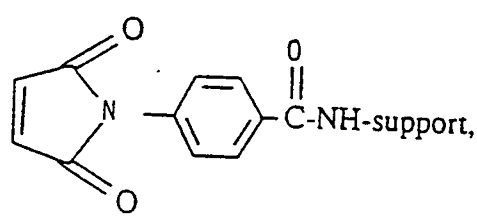

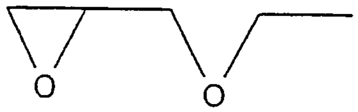 support solide,

ou OHC-support solide.

4. Procédé selon l'une quelconque des revendications 1 à 3, dans lequel la sonde de détection comprend un marqueur détectable et où la présence de la sonde de détection dans la fraction immobilisée séparée ou dans la solution restante est déterminée par mesure de ce marqueur.

5. Procédé selon la revendication 1, dans lequel le marqueur détectable est un groupe à activité enzymatique, un corps fluorescent, un chromophore, un corps luminescent, un radio-isotope ou un ligand à liaison spécifique, qui peut être déterminé par liaison avec un partenaire de liaison marqué.

6. Procédé selon l'une quelconque des revendications 1 à 5, dans lequel l'hybride formé entre la séquence polynucléotidique à laquelle on s'intéresse et la sonde de détection est antigéniquement distinct de ses brins individuels et dans lequel la présence de la sonde de détection dans la fraction immobilisée séparée est déterminée par fixation avec un réactif de type anticorps qui est sélectif pour la fixation dudit hybride et mesure du réactif de type anticorps qui s'est fixé à cette fraction immobilisée séparée.

7. Combinaison de réactifs pour la détection de la présence d'une séquence polynucléotidique particulière dans un échantillon d'essai, comprenant (1) une première sonde de séparation, (2) une seconde sonde de détection, lesdites sondes comprenant chacune au moins une séquence de bases monocaténaire qui peut s'hybrider avec une portion à exclusion réciproque de la séquence à détecter, la sonde de séparation comprenant de plus un groupe chimique réactif capable de former une liaison covalente stable avec un support solide réactif et (3) le support solide réactif, où le groupe chimique réactif dans la sonde de séparation est choisi parmi $—NH_2$, —SH, —COOH,

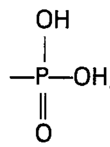, 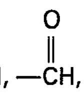, et —OH

et où le support solide réactif est choisi parmi

HO-support solide,

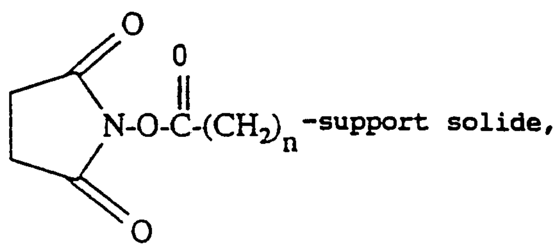

HS-support solide, 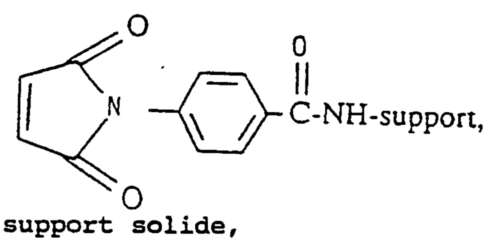

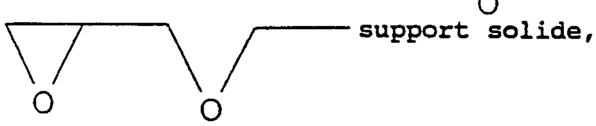

ou OHC-support solide.

8. Utilisation de la combinaison de réactif de la revendication 7 pour la détection de la présence d'une séquence polynucléotidique particulière dans en échantillon d'essai.

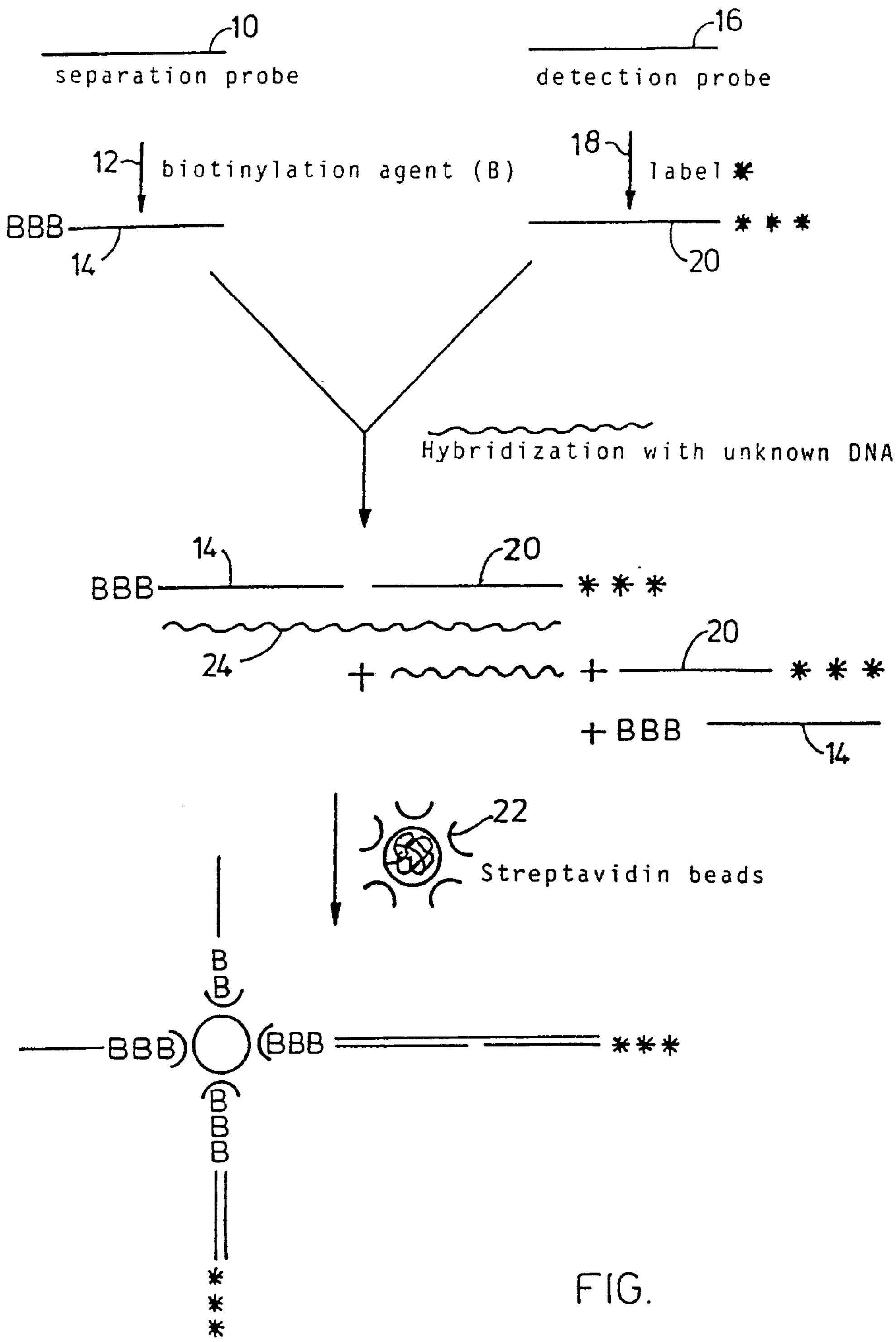
10
separation probe
16
detection probe
12
biotinylation agent (B)
18
label *
BBB
14
***
20
Hybridization with unknown DNA
14
20
BBB
***
24
+
+
20
***
+ BBB
14
22
Streptavidin beads
B
B
BBB
BBB
***
B
B
B
***

FIG.

MD 218